# EUROPEAN PATENT SPECIFICATION

(11) **EP 3 585 327 B1**
(45) Date of publication and mention of the grant of the patent: **11.08.2021**
(21) Application number: 18709865.2
(22) Date of filing: 22.02.2018
(51) Int. Cl.: A61F 5/56, A61M 16/06

(54) **A SYSTEM FOR SLEEP-DISORDERED BREATHING TREATMENT**
SYSTEM ZUR BEHANDLUNG VON SCHLAFGESTÖRTER ATMUNG
SYSTÈME DE TRAITEMENT DE TROUBLES RESPIRATOIRES DU SOMMEIL

(30) Priority: 24.02.2017 US 201762463425 P
(43) Date of publication of application: 01.01.2020
(73) Proprietor: Somnifix International LLC, Washington, District of Columbia 20017 (US)
(72) Inventor: MICHALAK, Nicholas, McLean Virginia 22102 (US); MICHALAK, Andre, McLean Virginia 22102 (US)
(74) Representative: HGF
(86) International application number: PCT/US2018/019226
(87) International publication number: WO 2018/156762

(56) References cited:
- US-A1- 2014 360 502
- US-A1- 2017 028 162

## Description

### BACKGROUND

Document US2017/028162 A1 discloses devices and methods for inhibiting mouth breathing and/or improving nasal breathing. Document US2014/360502 A1 discloses a closure for use during Positive Airway Pressure treatment of Obstructive Sleep Apnea for controlling air leakage through the lips and mouth of a person.

It is known that some individuals suffer from mild to severe sleep disorders. Some individuals merely have difficulty falling asleep, or perhaps staying asleep, while others have difficulty maintaining consistent, healthy breathing patterns. Individuals who suffer from sleep apnea are known to regularly stop breathing for 10 seconds or more, and in some cases, the apnea can last for minutes. This can cause a variety of problems for the individual, including drowsiness, irritability, cardiovascular deficiencies and diseases, and cognitive deficiencies and diseases.

Additionally, it is known that sleep apnea can be difficult to initially detect, and can also be difficult to effectively treat. Frequently, individuals suffering from sleep apnea are unaware of experiencing apneas throughout the night, and are unaware of what is causing their drowsiness the next day. Once a formal sleep study is conducted, diagnosis of sleep apnea can be made.

Treatments after diagnosis often include lifestyle changes, medication, and behavioral therapy. Additionally, in some cases, a continuous positive air pressure (CPAP) machine can be used and a splint employed in order to facilitate and encourage healthier breathing patterns during sleep. Another common treatment method for sleep apnea is oral appliance therapy (OAT), which uses a mandibular advancement device (MAD) to hold the jaw in a position that precludes airway collapse.

These approaches which involve the use of a CPAP machine or MAD are designed to be used by themselves without the need of additional components or devices used in conjunction with them.

### SUMMARY

There is a need for a device capable of externally maintaining the lips and/or jaw of the face of a user or patient, such as a patient diagnosed with a sleep disorder, in a relaxed, closed posture, in order to facilitate respiration through the nasal passages and aid in the treatment and prevention of sleep apnea or related sleeping disorders. Such a device would augment the efficiency of some of these treatments, particularly the CPAP and MAD treatments, by correcting the deficiencies in the previous approaches.

The present disclosure relates to a combination of a sleep strip (e.g., face sleep strip) designed to be worn by a person during sleep and a device for treating sleep-disordered breathing (SDB). More specifically, the present disclosure relates to a combination of a sleep strip configured to cover a mouth and lips of a user or patient and a continuous positive airway pressure device configured to continuously blow pressurized air into the nasal passages of the user or patient or a mandibular advancement device configured to hold a jaw of the user or patient in position. The object of the present disclosure is to ensure healthy breathing patterns in the user or patient, specifically by preventing breathing patterns that induce sleep apneas or snoring and by ensuring that the user or patient's airway does not collapse, thus ensuring the user or patient can breathe through the nasal passages.

The following presents a general summary of aspects of the present disclosure in order to provide a basic understanding of the disclosure. This summary is not an extensive overview of the disclosure and is not intended to identify key or critical elements of the present disclosure or to delineate the scope of the present disclosure. The following summary merely presents some concepts of the present disclosure in a general form as a prelude to the more detailed description provided below. This invention is defined by the independent claim. Preferred embodiments are defined in the dependent claims.

The present disclosure relates to a combination of a sleep strip configured to hold a user or patient's mouth in a naturally closed position such that the tongue and jaw of the user or patient will not relax during sleep and thereby cause pauses in breathing and a CPAP machine or MAD. The sleep strip includes a hypoallergenic adhesive affixed to a paper-thin, perforated silicone or cloth (e.g., polyester) mesh layer such that the sleep strip is securely and temporarily affixed to the face of a user or patient (e.g., the cheeks, lips, and/or jaw of the user or patient). The sleep strip according to the present disclosure is configured to hold the lips of the user or patient closed such that the user or patient can breathe through the nasal passages and nostrils while aided by the CPAP machine or MAD. A sleep "mask" is disclosed in U.S. Patent Serial No. 8,991,399 and in U.S. Patent Serial No. 9,795,175.

The sleep strip includes a first layer having an opening configured to provide an alternate channel for oral respiration during use of the sleep strip in an event that one or more nasal passages of the user or patient become blocked, forcing the user or patient to breathe through the mouth. The opening is disposed on a location on the first layer and is configured to lie over or just above the mouth of the user or patient during use of the sleep strip. The sleep strip also includes a permeable mesh layer, which includes a mesh disposed over the first layer such that the permeable mesh layer covers at least a portion of a surface of the first layer and covers the opening in the first layer.

The sleep strip is configured to conform to the shape of the user or patient's face, and provide a semi-secure seal over the mouth of the user or patient during use. While the seal need not be completely air-tight, any restriction over the mouth of a user or patient during sleep will help to curtail his or her use of the mouth to breathe.

When the sleep strip is properly positioned and affixed to the face of the user or patient, the user or patient's respiration is channeled, and therefore encouraged to flow through the nasal passages of the user or patient rather than through the mouth. Through this method of respiration, it is less likely for the user or patient to suffer from a sleep disorder such as sleep apnea.

A method is also disclosed for treating sleep-disordered breathing in a patient includes prior to sleeping, placing a sleep strip on a face of the patient, the sleep strip being configured to maintain lips and/or a jaw of the patient in a closed posture, and the sleep strip including a first layer comprising an adhesive configured to attach the sleep strip to the face of a patient, the first layer having an opening extending therethrough, the opening being located above the lips of the patient, and a mesh layer covering at least the opening of the first layer, the mesh layer being configured to allow air to flow therethrough; and disposing a device configured to facilitate stable breathing patterns on a face or in a mouth of the patient.

According to one aspect, the method is for treating sleep apnea, and the device is a continuous positive air pressure machine.

According to one aspect, the method is for treating sleep apnea, and the device is a mandibular advancement device.

According to one aspect, the method is for treating snoring, and the device is a continuous positive air pressure machine.

According to one aspect, the method is for treating snoring, and the device is a mandibular advancement device.

According to one aspect, the sleep strip is releasably secured only onto the patient's lips.

According to the invention, the mesh layer extends across an entirety of a front side of the first layer.

According to one aspect, the adhesive is located around an entire border of the opening.

According to one aspect, the device is a continuous positive air pressure machine that is disposed on the face of the patient over at least a portion of the sleep strip.

According to one aspect, the device is a mandibular advancement device that is disposed on the face of the patient under the sleep strip.

In some embodiments of the present disclosure, a system for treating sleep-disordered breathing in a patient includes a system for treating sleep-disordered breathing in a patient including a sleep strip configured to maintain lips and/or a jaw of the patient in a closed posture, the sleep strip including a first layer comprising an adhesive configured to attach the sleep strip to a face of a patient, the first layer having an opening extending therethrough, the opening being configured to be located above the lips of the patient when the sleep strip is attached to the face of the patient, a mesh layer, and a device configured to facilitate stable breathing patterns in the patient during a sleep session, wherein the mesh layer extends across an entirety of a surface of the first layer and covers the opening, but is configured to allow air to flow therethrough.

According to one aspect, the sleep strip also includes a release tab configured to facilitate removal of the sleep strip from the face of the patient after the sleep session is completed.

According to one aspect, the sleep strip is configured to be releasably secured onto the lips of the patient.

According to one aspect, the sleep strip is configured to be releasably secured only onto the lips of the patient.

According to the invention, the mesh layer extends across an entirety of a surface of the first layer.

According to one aspect, the device is a continuous positive air pressure machine.

According to one aspect, the device is a mandibular advancement device.

According to one aspect, the adhesive is located around an entire border of the opening.

A method for treating sleep apnea in a patient is also disclosed. The method includes the steps of placing, prior to sleeping, a first device including a sleep strip on a face of a patient diagnosed with sleep apnea; disposing a second device configured to treat sleep apnea in connection with the patient; and maintaining stable breathing patterns in the patient.

A method for treating snoring in a patient is also disclosed. The method includes the steps of placing, prior to sleeping, a first device including a sleep strip on a face of a patient diagnosed with snoring; disposing a second device configured to treat snoring in connection with the patient; and maintaining stable breathing patterns in the patient.

### BRIEF DESCRIPTION OF THE DRAWINGS

FIG. 1A is a side view of a MAD in the mouth of a user while the user's lips are in a closed position.
FIG. 1B is a side view of a MAD in the mouth of the user while the user's lips are in an open position.
FIG. 1C is a side perspective view of a MAD in the mouth of the user while the user's lips are in an open position.
FIG. 2A is a side perspective view showing a nasal mask CPAP machine on the face of a user.
FIG. 2B is a front perspective view showing a nasal pillow CPAP machine on the face of a user.
FIG. 2C is a side perspective view showing a full face mask CPAP machine on the face of a user.
FIG. 3 is a front perspective view of a sleep strip according to one exemplary embodiment.
FIG. 4 is an exploded assembly view of a rear side of the sleep strip shown in FIG. 3.
FIG. 5 is an exploded assembly view of a front side of the sleep strip shown in FIG. 3.
FIG. 6 is a perspective view of a rear surface of the sleep strip according to one aspect.
FIG. 7 is a perspective view of a rear surface of the sleep strip according to another aspect.
FIG. 8 is a rear perspective view of a sleep strip according to an alternative embodiment.
FIG. 9 is a rear perspective view of a sleep strip according to another alternative embodiment.
FIG. 10A is a side perspective view of a system for treating sleep-disordered breathing including a nasal mask CPAP machine and a sleep strip.
FIG. 10B is a side perspective view of a system for treating sleep-disordered breathing including a MAD and a sleep strip.
FIG. 11 is a flowchart illustrating a method for treating sleep apnea in a patient.
FIG. 12 is a flowchart illustrating a method for treating snoring in a patient.

### DETAILED DESCRIPTION

The systems and methods according to the present disclosure are capable of embodiments in many different forms, and there are shown in the drawings, and will herein be described in detail, certain embodiments with the understanding that the present disclosure is to be considered exemplary of the principles of the invention and is not intended to limit the broad aspects of the invention to the embodiments illustrated and described.

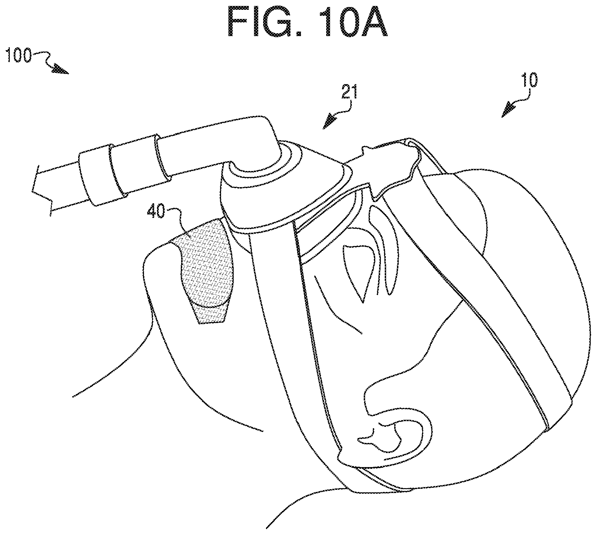

Referring generally to the FIGURES, the present disclosure relates to a system, such as system 100 shown in FIGS. 10A-10B, for treating sleep disorders such as obstructive sleep apnea (OSA) and/or snoring. The system 100 includes a first device including a sleep strip configured to be worn on the face of a user or patient during sleep in combination with a second device suitable for treating some of the phenomena associated with OSA or related sleep disorders such that any deficiencies in the operation of the second device are substantially corrected. In some examples, the second device is a CPAP machine. In some additional examples, the second device is a MAD. Various exemplary embodiments are described below.

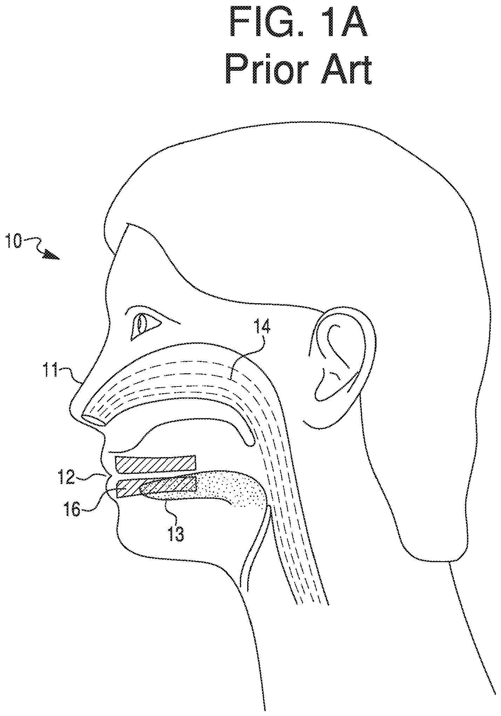

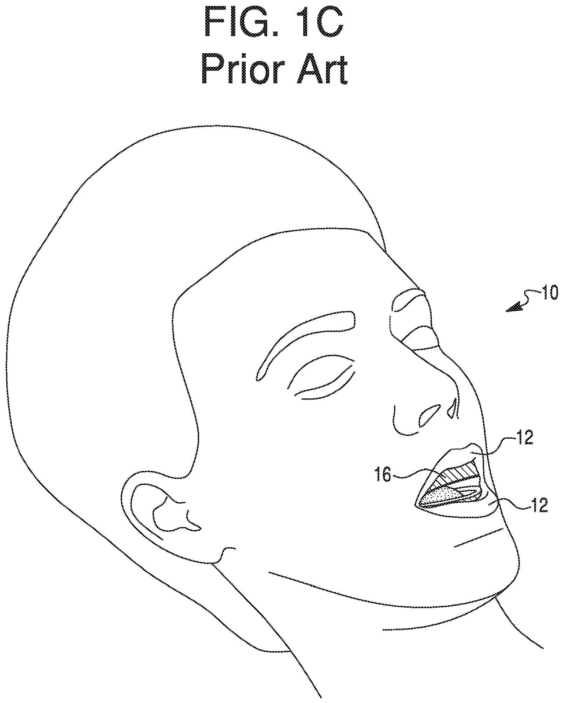

As shown in FIGS. 1A-1C, a MAD 16 is an oral appliance apparatus configured to treat snoring and OSA by inserting the MAD 16 into the mouth of a user or patient 10 and keeping the MAD 16 in the mouth of the user or patient while the user or patient 10 sleeps. A MAD 16 is generally made of a hard acrylic material, although in some cases, a MAD 16 is made of a softer material. A MAD 16 is customizable to fit the upper and lower teeth and/or jaws of a particular user or patient and is configured to hold the lower jaw of the user or patient 10 slightly forward from its natural position such that the user or patient's airway remains open during sleep.

A MAD 16 may be formed of a variety of materials and configured to be disposed in variation locations in the mouth of a user or patient. For example, a PM Positioner™ is formed of an acrylic material and has an adjustment mechanism disposed on a cheek side of the device near a user or patient's molars when the device is worn by the user or patient. As another example, the SomnoDent® SUAD™ is formed of an acrylic material reinforced by a metal framework and has an adjustment mechanism which includes two elastic bands and which keeps the jaw of the user or patient from falling open during sleep. As another example, a SomnoDent® MAS device is formed of an acrylic material and has a recessed screw mechanism on each side of an upper part and ball clasps which hold the device in position. As another example, an EMA® (Elastic Mandibular Advancement) device is formed of a plastic material and has an upper part and a lower part connected to the upper part by rubber straps on sides of the device; by using differently sized straps, the device may be positioned differently while in use. As another example, a TAP® (Thornton Adjustable Positioner) is a device formed of a nickel-free, allergen-free material reinforced by a cobalt-chromium hardware and has a hook and socket mechanism which holds an upper tray and a lower tray together; the device's position is adjustable. As another example, a Herbst Appliance is formed of acrylic, thermal active, and soft materials and has a post and sleeve mechanism to advance a jaw of a user or patient and a ball and hook mechanism such that a vertical opening degree is adjustable.

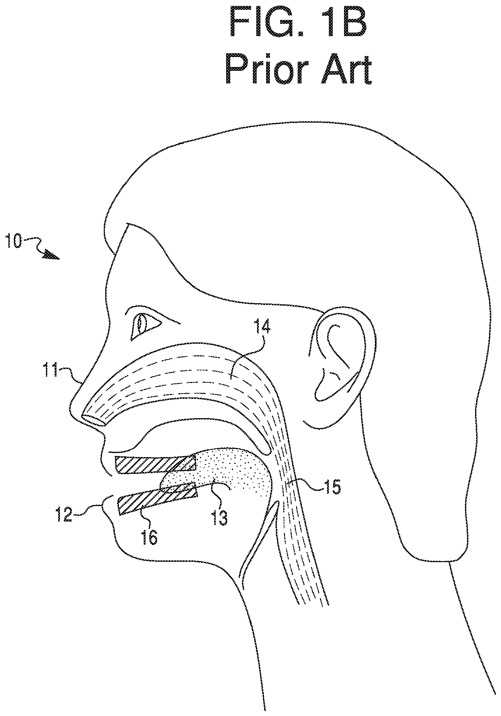

However, a MAD 16 is ineffective if a tongue 13 of a user or patient 10 does not remain sealed to the palate. Because it is often difficult for the user or patient 10 to naturally close the mouth over a MAD 16, users or patients often experience lip incompetence. Lip incompetence leads to an oral respiratory breathing pattern that results in a loss of a seal between the tongue 13 and palate of the user or patient. Once the seal is lost, the tongue 13 falls back into the airway; the blocked airway results in snoring and sleep apnea, as is shown in FIG. 1B.

In FIG. 1A, a MAD 16 is shown in place in the mouth of a user or patient 10 with lips 12 of the user or patient 10 in a closed position. A tongue 13 of the user or patient 10 is in a position in the mouth of the user or patient 10 (e.g., the tongue 13 is pressed to the palate) such that the user or patient's airway 14 remains open and the user or patient 10 can breathe through nostrils 11. FIGS. 1B-1C show a MAD 16 in place in the mouth of the user or patient 10 with the lips 12 of the user or patient 10 in an open position. A user or patient 10 has difficulty maintaining lip competence while using the MAD 16 because it is difficult for the user or patient 10 to naturally close the lips 12 over the MAD 16. When the lips 12 are in an open position, the tongue 13 relapses to airway 14 causing a lower portion 15 of the airway 14 to become blocked, rendering it difficult or impossible for the user or patient to maintain a stable breathing pattern through the nostrils 11. The result is snoring and/or OSA or related sleep disorders. Thus, during use of the MAD 16, opening the lips 12 of the user or patient 10 may lead to snoring or sleep apneas because of the tongue 13 relapse and subsequent obstruction of the airway of the user or patient 10. Unless a patient using the MAD 16 has an individual who can inform the user or patient 10 of snoring or sleep apneas, the user or patient 10 will be oblivious to poor sleep.

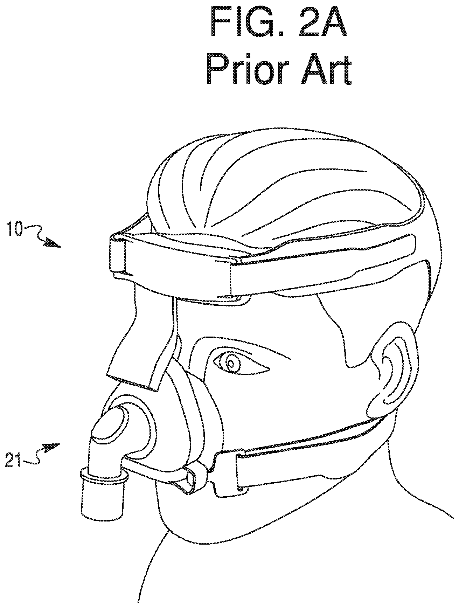

Referring now to FIGS. 2A-2C, a CPAP machine continuously blows pressurized air into nasal passages of a user or patient 10 to ensure that an airway 14 (shown in FIGS. 1A-1C) of the user or patient 10 does not collapse or close. Depending on the degree of airway collapsibility for a particular patient, a CPAP machine may deliver varying amounts of pressurized air; for example, a patient with a relatively severe airway collapsibility will have a higher pressurization than does a patient having a relatively low airway collapsibility. The amount of pressurized air is titrated during a polysomnography (PSG) until the airway 14 remains patent, that is, open and unobstructed.

There are three different approaches to a CPAP machine. First, a nasal mask, such as nasal mask CPAP machine 21 (shown in FIG. 2A), which is configured to cover the nose only, may be used. The nasal mask 21 is of a triangular shape and has a silicone cushion. The nasal mask 21 is desirable for high-pressure air delivery, but it is ineffective for mouth breathers or those with temperature-induced allergies. Of the three approaches, the nasal mask 21 is the most popular option for a CPAP machine.

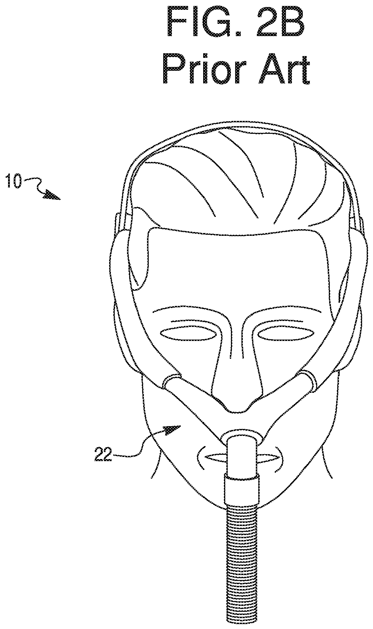

Second, a nasal pillow, such as nasal pillow CPAP machine 22 (shown in FIG. 2B), which is a plastic insert configured to fit directly into the nostrils of a user or patient 10, may be used. The nasal pillow 22 creates an optimal seal, so it is desirable for relatively lower pressure delivery. The nasal pillow 22 is ineffective for mouth breathers and may cause extreme nose dryness and/or pressure ulcers.

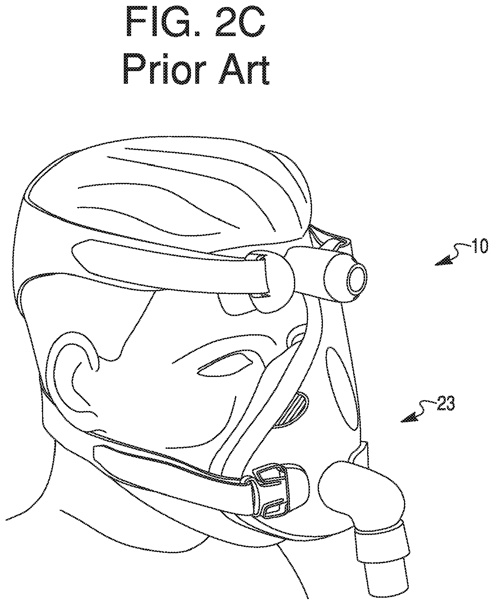

Third, a full face mask, such as full face mask CPAP machine 23 (shown in FIG. 2C), may be used. The full face mask 23 is currently the best option for mouth breathers. However, the full face mask 23 restricts movement, needs significantly higher air-pressure levels than either the nasal mask 21 or the nasal pillow 22 in order to operate effectively in maintaining desirable breathing patterns when the user or patient is sleeping. There is a low compliance rate for patients who use the full face mask.

When a user or patient 10 is using a CPAP machine, such as the nasal mask 21, the nasal pillow 22, or the full face mask 23, the user or patient 10 is aware when the CPAP machine is not keeping the user or patient's airway patent because leakage of air from the nose or mouth of the user or patient tends to wake the user or patient. Specifically, for nasal mask users, opening the mouth causes pressurized air to enter the nose and exit the mouth of the user or patient. A noticeable turbulence will wake the user or patient 10 and cause the user or patient 10 to close the mouth to continue receiving the pressurized air in the airway. Alternatively, the user or patient 10 may remove the nasal mask 21, the nasal pillow 22, or the full face mask 23 entirely, rendering treatment by a CPAP machine moot.

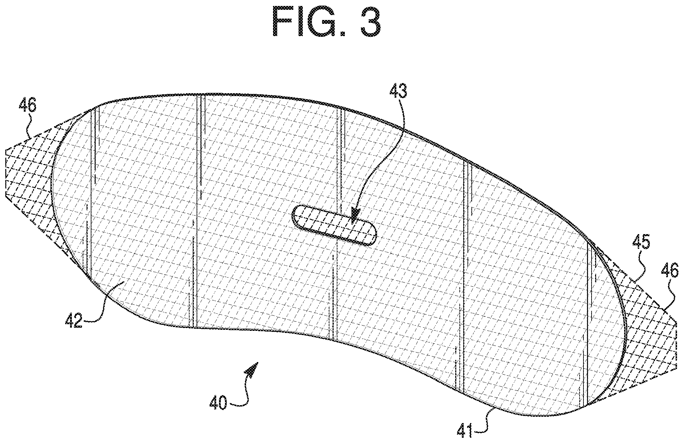

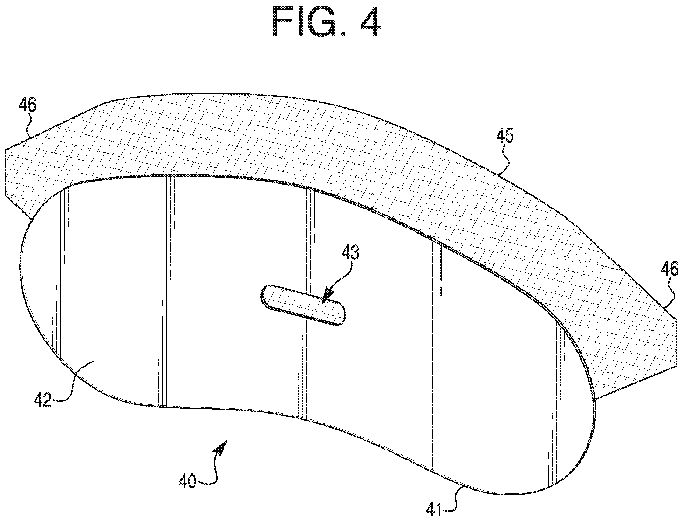

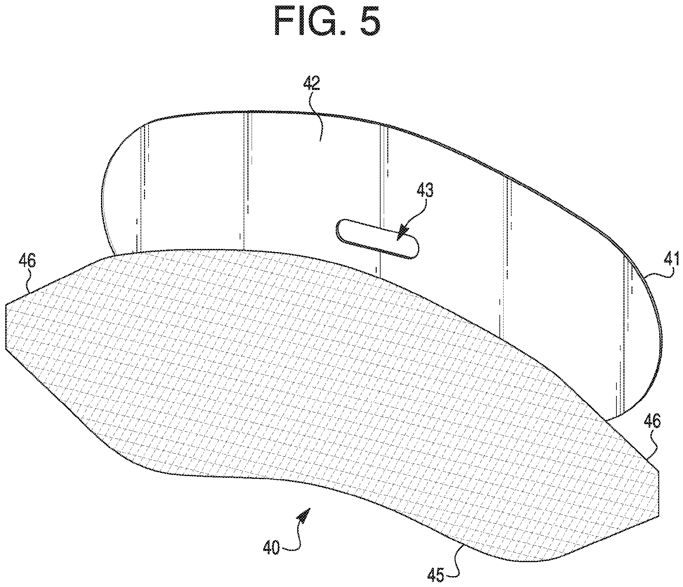

As shown in FIGS. 10A-10B, the system 100 also includes a sleep strip 40. As can best be appreciated with reference to FIGS. 3-5, the sleep strip 40 is a device configured to be worn on a face of a user or patient during sleep. For example, the sleep strip 40 is a device configured to be securably and/or releasably attachable to a mouth and/or lips of the user or patient. In one example, the sleep strip 40 is configured to be securably and/or releasably attachable only to the lips of the user or patient. According to one specific example, the sleep strip 40 is configured to be securably and/or releasably attachable to at least a portion of the lips of a user or patient, such as the vermillion of the upper lip or the vermillion of the lower lip of the user or patient. As an additional example, the sleep strip 40 is configured to be securably attachable only to an external portion of the lips or mouth of the user or patient. As an additional example, the sleep strip 40 is configured to be securably and/or releasably attachable to an internal portion and/or an external portion of the lips or mouth of the user or patient. According to another specific example, the sleep strip 40 is configured to be securably attachable over, under, and/or around the upper lip and the lower lip of the user or patient. In one example, the sleep strip 40 is configured to be securably attachable both to the upper lip and the lower lip of the user or patient and to a region of the face of the user or patient extending to about 2 mm beyond the edges of the upper lip and the lower lip of the user or patient.

Still referring to FIGS. 3-5, the sleep strip 40 includes a first layer 41 including an adhesive. The first layer 41 has a front surface 42 and a rear surface 44. In some aspects, the sleep strip 40 is formed of a flexible material such that the sleep strip 40 has a plastic response when a mechanical stress is placed and then subsequently removed from the first layer 41 (e.g., when the stress is removed, the mesh layer 45, as described below, returns to its original shape and/or orientation). The first layer 41 of the sleep strip 40 is configured to ensure that a user or patient's lips are held in position such that the upper and lower lips do not separate when the user or patient is wearing the sleep strip 40.

A front surface 42 of the first layer 41 is configured to adhere to a face of a user or patient such that the sleep strip 40 fits comfortably on the lips, mouth, and/or jaws of a user or patient without placing an external stress on the user or patient's mouth or jaw. A rear surface 44 of the first layer 41 is configured to adhere to a mesh layer 45 (described in more detail below). The sleep strip 40 is configured to ensure that a user or patient does not rely fully upon oral respiration during sleep, as oral respiration predisposes the user or patient to snoring and/or apnea.

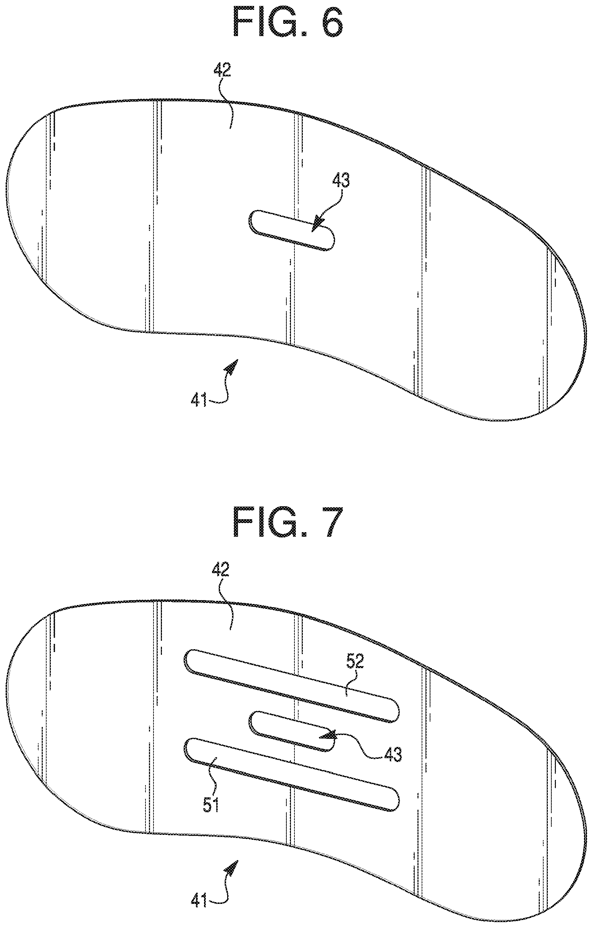

The front surface 42 of the first layer 41 of the sleep strip 40 is equipped with an adhesive substance which is sufficiently strong to bind the sleep strip 40 temporarily to skin, yet does not irritate the skin or leave a sticky residue on the skin. According to one aspect, the adhesive covers an entire surface of the front surface 42, as shown in FIG. 6. According to another aspect, the adhesive covers only a portion of the front surface 42, shown as adhesive strips 51 and 52 in FIG. 7. The shape of adhesive strips 51 and 52 is any suitable shape. In one example, the adhesive is clear. In another example, the adhesive has a slight coloration to facilitate a user or patient in differentiating between the first layer 41 and the mesh layer 45 of the sleep strip 40. According to one aspect, the adhesive is capable of remaining in position even in the event that the user or patient begins to sweat. Additionally, according to another aspect, the adhesive of the sleep strip is non-toxic. The non-toxicity of the adhesive ensures that when a sleep strip 40 is applied to the lips and/or mouth of the user or patient, a toxic substance is not thereby introduced onto the lips or into the mouth of the user or patient. The adhesive of the sleep strip 40 is configured to affix the sleep strip 40 to the upper lip and lower lip and jaw of the user or patient. As one example, the adhesive of the sleep strip 40 is oriented just above and below the opening 43; for example, the adhesive is oriented around an entire border of the opening 43. As another, specific, example, the adhesive is configured to securably attach the sleep strip 40 to at least one of the upper vermillion and the lower vermillion of the lips of the user or patient.

The first layer 41 has at least one opening (e.g., permeation) 43 configured to allow air to pass through the sleep strip 40. The opening 43 is also configured to prevent a user or patient from engaging in inefficient oral respiratory patterns. However, it is desirable that a size and/or number of the opening 43 not be so high as to allow a user or patient to subconsciously engage in oral respiration. In some aspects, the opening 43 is positioned on the sleep strip 40 in a configuration such that when the sleep strip is worn by the user or patient, the opening 43 lies directly above and/or around the lips of the user or patient. In some aspects, the opening 43 is positioned at a central position on the first layer 41.

Entry and exit points of a nasal cavity (i.e., the two nostrils) of a patient generally have an area of 350 square millimeters, and humans generally use one nostril when breathing nasally. The relatively small size of the two nostrils assists in optimizing a CO₂-O₂ exchange, as air is recycled, allowing more time to extract maximum O₂. On the other hand, the entry and exit point of the oral cavity (i.e., the mouth) is significantly larger. When a person breathes orally, the person may exacerbate airway collapsibility by exhaling all of the air in the respiratory airway upon exhalation, decreasing the amount of O₂ retained.

The opening 43 allows for oral respiration if nasal respiration becomes impossible. The opening 43 is configured to conform to a user or patient's nostril area, nasal cavity, and/or oral cavity. The opening 43 may mimic the nostrils, with the mesh layer 45 acting as a filter, and the small size of the opening 43 functioning as a check valve to preclude full oral exhalation, and thereby improve CO₂-O₂ exchange. The opening 43 is purposely small to encourage nasal respiration. In the case of full nasal blockage and difficulty with oral respiration, a user or patient or another person may remove the sleep strip from the face of the user or patient. This removal ability reduces or prevents the possibility of hypercapnia, which occurs when there are excessive levels of CO₂ in the blood.

The opening 43 exists to provide a restricted channel for air to pass through. The opening 43 has a cross-sectional area, according to one example, no larger than about 125 square millimeters and may have any suitable geometry. For example, the opening 43 may have a square cross-sectional shape. As an additional example, the opening 43 may have a rectangular cross-sectional shape. As another example, the opening 43 may have a circular cross-sectional shape. As yet another example, the opening 43 may have a somewhat elliptical cross-sectional shape as shown in FIGS. 3-5. Because the opening 43 is covered by the mesh layer 45, the sleep strip 40 is configured to provide a constricted, alternate passage for air in the event that the user or patient's nasal passage becomes clogged, and alternate breathing through the mouth is required. In some aspects, the dimensions of the opening 43 (in a lateral orientation) is a height of between about 2.5 to about 5 millimeters and a width of between about 12.5 to about 19 millimeters. In some aspects, the opening has a height of about 3 millimeters and a width of about 16 millimeters. In some aspects, the opening measures about 50 square millimeters.

According to one example, the first layer 41 of the sleep strip 40 is covered with an adhesive backing, such as a piece of waxed paper, in order to keep the first layer 41 (for example, the adhesive on the front surface 42 of the first layer 41) clean and sterile until time of use. Similarly, in some exemplary embodiments, such as those sized for smaller mouths or faces, the adhesive of the sleep strip 40 may also encompass or come into contact with the opening 43 or come very near to the site of the opening 43 of the sleep strip 40. The adhesive of the first layer 41 is also configured to leave little to no sticky residue behind, and is capable of holding a stable position on the face of the user or patient that endures for the duration of the sleep session.

Referring back to FIGS. 3-5, the sleep strip 40 also includes the mesh layer 45. The mesh layer 45 is formed of a paper-thin cloth, a silicone sheet, a woven paper, or similarly soft material. As one example, the mesh layer 45 is formed of polyester. The mesh layer 45 is disposed in contact with the rear surface 44 of the first layer 41. The mesh layer 45 is configured to be stretchable, yet strong enough not to break or puncture when stretched. The mesh layer 45 is also configured to be gas-permeable such that the mesh layer 45 provides an alternative passage for air to reach the user or patient and thereby allow for oral respiration by the user or patient in an emergency (for example, when the nasal passages of the user or patient become clogged, rendering nasal respiration difficult or impossible). According to the invention, the mesh layer 45 extends across an entirety of the rear surface 44 of the first layer 41, including the opening 43.

Referring still to FIGS. 3-5, in some embodiments, the sleep strip 40 includes at least one release tab 46 connected to the mesh layer 45. As shown in FIGS. 3-5, the sleep strip 40 includes two release tabs 46, but the sleep strip 40 is not limited to this particular implementation. The release tab 46 is positioned, for example, on a left or a right side of the mesh layer 45 or on both the left and right sides of the mesh layer 45, and is free from adhesive. The release tab 46 is designed to facilitate the removal of the sleep strip 40 from the face of a user or patient after the sleep session is completed. To use the release tab 46, the user or patient simply pulls the tab 46 out and away from the user or patient's face, providing for the sleep strip 40 to be peeled off the face of the user or patient quickly and easily.

It is envisioned that the sleep strip 40 is capable of remaining securely affixed to or over the lips and jaw of the user or patient for the duration of the sleep session due to the strength and rigidity of the material used to form the mesh layer 45, as well as due to the efficacy of the first layer 41 of the sleep strip 40. It should be noted that the first layer 41 and the mesh layer 45 of the sleep strip 40 are hypoallergenic and may be kept in a sterile container until use. The sleep strip 40 may also be kept in a sterile polypropylene envelope, similar to a conventional adhesive bandage, in order to ensure dirt and other contaminants do not compromise the sterility and/or cleanliness of the sleep strip 40.

Alternate aspects of the sleep strip 40 according to the present disclosure may include variations on the size of the opening 43. For example, for users or patients with a large lung capacity, the standard size of the opening 43 of the sleep strip 40 may be too small, which could cause the user or patient to faint from a lack of oxygen if the user or patient's nasal passages become blocked during the use of the sleep strip. As such, according to some aspects, the sleep strip 40 includes an opening 43 that has a larger diameter, or if the opening 43 is a mesh screen, the mesh screen may be larger or equipped with additional small holes to permit slightly greater air flow.

Similarly, the sleep strip 40 may be available in a variety of sizes in order to accommodate all shapes and sizes of faces. While it is envisioned that a 'one-size-fits-all' size of the sleep strip 40 will likely function appropriately for most individuals, larger sizes may be needed for individuals with irregularly shaped or sized mouths or other facial features.

In the event that the user or patient has a full face beard, the adhesive of the sleep strip 40 will be less effective than when placed on a user or patient without facial hair. As such, it is envisioned that an alternate embodiment of the sleep strip 40 includes at least one elastic band (not shown) which may facilitate the stable placement of the sleep strip 40 onto the user or patient's face. It is envisioned that the elastic band would be affixed to the sides of the sleep strip 40 and be capable of retaining the sleep strip 40 in position on the mouth and/or lips of the user or patient by extending the elastic band around the user or patient's ears or the back of the user or patient's head. An adhesive may still be employed in this alternate embodiment of the sleep strip 40; however, it is understood that facial hair would likely infringe on the adhesive's capacity to remain affixed to the face, mouth, and/or lips of the user or patient for the duration of the sleep session. An alternate embodiment of the sleep strip 40 may be a smaller version of the sleep strip 40. It is envisioned that the smaller version would interact only with the lips of the user or patient, to ensure that no facial hair would infringe on the adhesive.

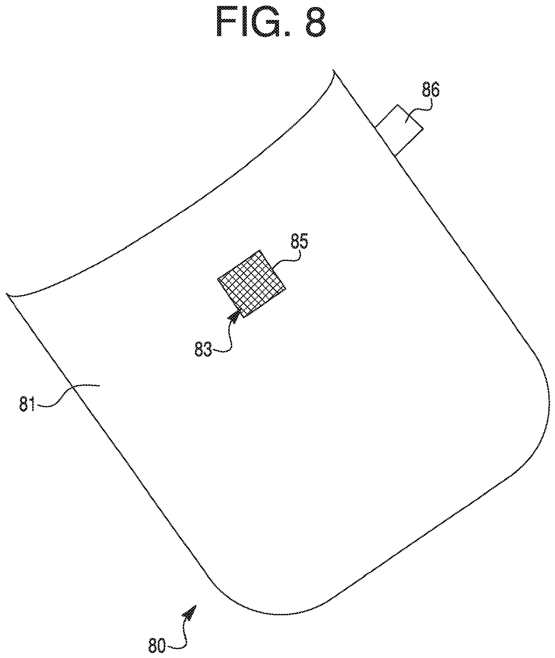

Referring now to FIG. 8, another exemplary embodiment of the present disclosure relates to a sleep strip 80. The sleep strip 80 is as the sleep strip 40 herein disclosed, except for the differences described below. The sleep strip 80 is of a different size and/or shape compared to the sleep strip 40, as can be seen by comparing FIG. 3 and FIG. 8. The sleep strip 80 includes a first layer 81 which is as the first layer 41 described above. The sleep strip 80 also includes a release tab 86 configured to allow a user or patient to remove the sleep strip 80 from the face, mouth, and/or lips of the user or patient. The sleep strip 80 also includes an opening (e.g., permeation) 83 which may be as opening 43 of the sleep strip 40 described above. As shown in FIG. 8, the opening 83 includes a mesh 85 disposed only within the opening 83 of the sleep strip 80. The opening 83 of the sleep strip 80 as shown in FIG. 8 has a rectangular cross-section (for example, a square cross-section).

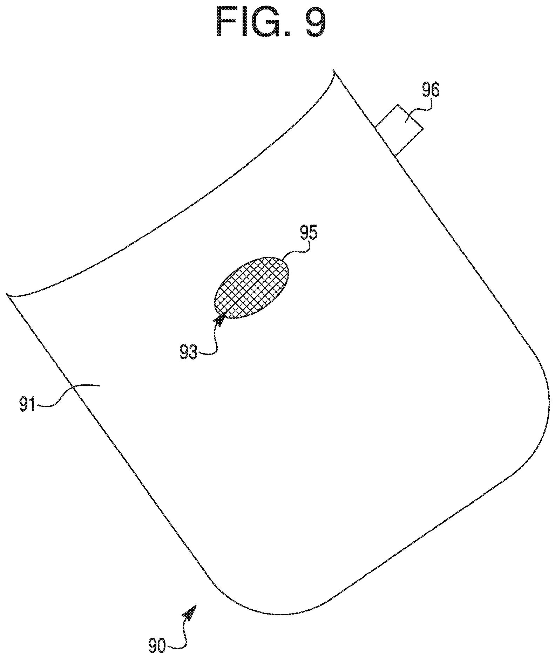

Referring now to FIG. 9, another exemplary embodiment of the present disclosure relates to a sleep strip 90. The sleep strip 90 is as the sleep strip 80 herein disclosed, except for the differences described below. The sleep strip 90 includes a first layer 91 which is as the first layer 41 described above. The sleep strip 90 also includes a release tab 96 configured to allow a user or patient to remove the sleep strip 90 from the face, mouth, and/or lips of the user or patient. The sleep strip 90 also includes an opening (e.g., permeation) 93 which may be as opening 43 of the sleep strip 40 described above. As shown in FIG. 9, the opening 93 includes a mesh 95 disposed only within the opening 93 of the sleep strip 90. The opening 93 of the sleep strip 90 as shown in FIG. 9 has an elliptical cross-section (for example, a circular cross-section).

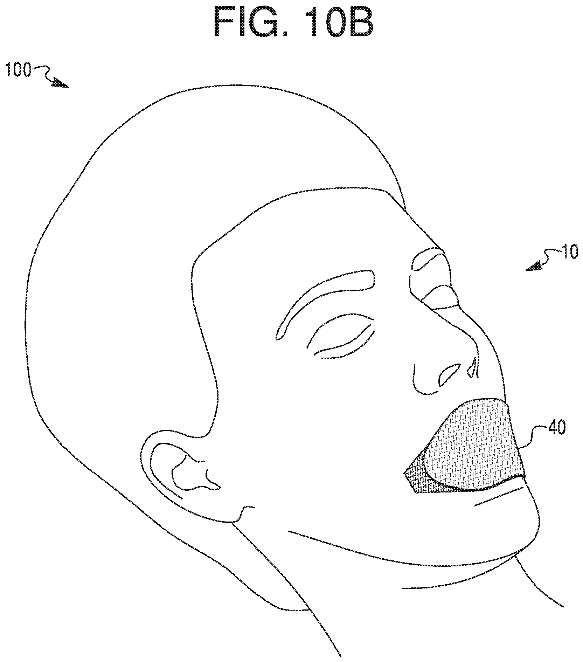

As shown in FIGS. 10A-10B, in some embodiments, a system 100 for preventing sleep disordered breathing in a patient (such as breathing associated with a sleep disorder, e.g., sleep apnea, snoring, etc.) includes a sleep strip 40 as described above, including a mesh layer 45 and an first layer 41 with an opening 43 configured to allow air to permeate through the mesh layer 45 and also configured to attach the sleep strip 40 to a face of a user or patient 10. The system 100 also includes a second device configured to maintain stable breathing patterns for a user or patient during sleep. In some aspects, the second device is a CPAP machine, such as the nasal mask 21, as shown in FIG. 10A. In some additional aspects, the second device is a MAD, such as the MAD 16. FIG. 10B shows a user or patient 10 with a MAD inside the mouth (not shown) and a sleep strip 40 covering the mouth of the user or patient 10.

Clinical trials using the system 100 including a sleep strip 40 and a second device (such as a CPAP machine or a MAD) were carried out to assess the effectiveness of the system 100 at treating and/or preventing sleep disorders such as sleep apnea and/or snoring. The clinical trials were designed to test the hypothesis that the system 100 improves sleep apnea severity, measured by a apnea-hypopnea index (AHI), and reduces the severity of pharyngeal collapse relative to treatment of patients that does not include use of the sleep strip 40 disclosed herein (for example, no treatment for sleep disorders or treatment using only a CPAP machine or MAD). The clinical trials included 36 subjects (including expected trial dropout subjects). Subjects were determined based on the following inclusion and exclusion criteria. The inclusion criteria included: age of a subject ranged between 21 to 70 years; the subject had an AHI of between 10 to 50 events per hour; the subject had a body-mass index of less than 38 Kg/m²; and the subject had a neck circumference of less than 20 inches for men and less than 17 inches for women. The exclusion criteria included: any medication taken by a potential subject which is known to influence breathing, sleep/arousal or muscle physiology; concurrent sleep disorders (such as insomnia, narcolepsy, central sleep apnea, and parasomnia) in a potential subject; inability of a potential subject to sleep supine; and failure of a potential subject to comfortably breathe nasally for a period of 1 to 2 minutes.

The clinical trials included a regime of four home sleep testing sessions conducted over the course of one month and determined in random order for each test subject: 1) a sleep session with no treatment; 2) a sleep session using only an oral appliance (such as a CPAP machine or MAD); 3) a sleep session using an oral appliance and a sleep strip; and 4) a sleep session using only a sleep strip. The test subjects were asked to maintain a sleep log. Additionally, sensors were attached to the bodies of the subjects for the duration of a sleep session. The sensors recorded electroencephalographic signals, electrocardiographic signals, nasal pressure, oxygen saturation, snoring decibel levels, and chest and abdominal movements. According to the clinical trials, the use of system 100 may result in a decrease of between 20% and 100% in the AHI. According to the clinical trial design, any decrease in AHI of 10 events per hour or more is considered to be clinically important.

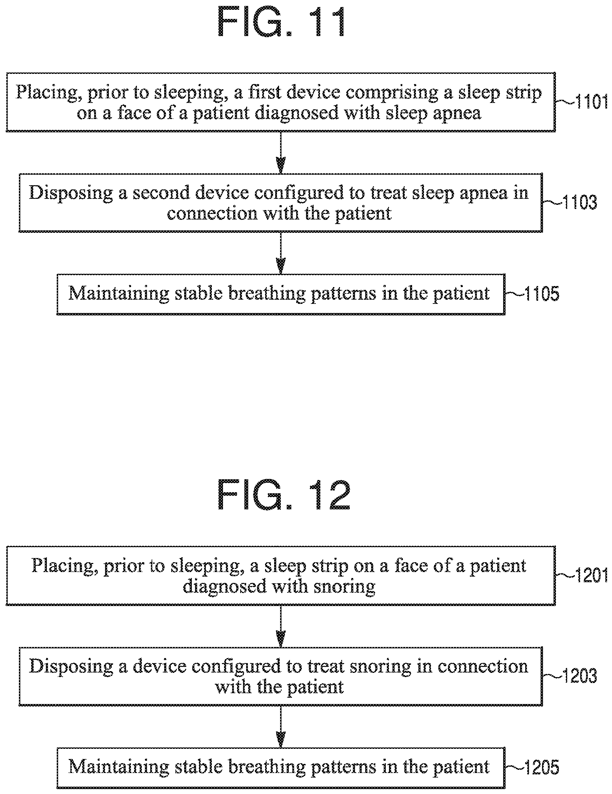

A method 1100, illustrated in FIG. 11, for treating sleep disorders (e.g., sleep apnea) is disclosed. The method 1100 includes the step 1101 of placing, prior to sleeping, a first device including a sleep strip on a face of a patient diagnosed with snoring. The method 1100 also includes the step 1103 of disposing a second device configured to treat snoring in connection with the patient. The method 1100 also includes the step of maintaining stable breathing patterns in the patient. The sleep strip used in the method 1100 may be the sleep strip 40, the sleep strip 80, or the sleep strip 90 described above.

In some aspects, the method 1100 includes a first step of diagnosing a patient who suffers from a sleep disorder (e.g., sleep apnea). For example, the first step is diagnosing a patient who suffers from OSA because of a blockage in the patient's airways. OSA causes a patient to stop breathing for a short period of time, and OSA can occur periodically during a patient's sleep session. OSA is more common in men, adults over the age of 65, patients who are overweight, and patients who have jaw problems (e.g., micrognathia, retrognathia, etc.), though in some cases, other patients (e.g., children, women, etc.) also suffer from OSA. There are various symptoms of OSA which are used by a health care provider to make an OSA diagnosis. Some symptoms are evident during a patient's sleep, such as unusually loud snoring (both intermittent and continuous), gasping or choking, pauses in breathing patterns, abnormally sudden body movements, restless tossing and turning, and frequent awakenings. Some symptoms are evident during a patient's waking periods, such as feelings of sleep deprivation, headaches shortly after awakening, dry or sore throat after awakening, fatigue during the day, drowsiness during the day, mood swings, memory loss, and inability to concentrate.

In some aspects, diagnosing a patient who suffers from a sleep disorder (e.g., sleep apnea) is conducted by a polysomnography (e.g., a monitored overnight sleep session at a sleep center).

In some aspects, the second device is a continuous positive air pressure machine.

In some aspects, the second device is a mandibular advancement device.

In some aspects, the method 1100 also includes positioning the sleep strip such that an opening in a first layer of the sleep strip lies directly above a mouth of the patient.

In some aspects, the method 1100 also includes affixing the sleep strip to an upper lip and a lower lip of the patient using an adhesive.

In some aspects, the method 1100 also includes covering a mouth of the patient with the sleep strip.

In some aspects, the method 1100 also includes forming a semi-secure seal over the mouth of the patient with the sleep strip.

In some aspects, the step 1103 of the method 1100 includes reducing an incidence of the sleep disorder (such as sleep apnea) in the patient below some predetermined threshold relative to an incidence of sleep apnea in the patient prior to any treatment for sleep apnea. In some aspects, the method 1100 may also include reducing an incidence of sleep apnea in the patient relative to treatment without using a sleep strip as disclosed herein. As one example, the method 1100 may include reducing an incidence of sleep apnea in a patient from between 20% and 100%. As a specific example, the method 1200 may also include reducing an incidence of sleep apnea in the patient by between 50% and 90% relative to the incidence of sleep apnea in the patient prior to treatment using the sleep strip disclosed herein. As a more specific example, the method 1100 may also include reducing an incidence in sleep apnea in a patient by 50% relative to treatment without using the sleep strip. As an additional specific example, the method 1100 may also include reducing an incidence in a sleep disorder (e.g., OSA) in a patient to a level of clinical insignificance.

A method 1200, illustrated in FIG. 12, for treating snoring is disclosed. The method 1200 for treating snoring in a patient includes the step 1201 of placing, prior to sleeping, a first device including a sleep strip on a face of a patient diagnosed with snoring. The method 1200 also includes the step 1203 of disposing a second device configured to treat snoring in connection with the patient. The method 1200 also includes the step 1205 of maintaining stable breathing patterns in the patient. The sleep strip used in the method 1200 may be the sleep strip 40, the sleep strip 80, or the sleep strip 90 described above.

In some aspects, the method 1200 for treating snoring includes a first step of diagnosing a patient who suffers from snoring. For example, the first step includes diagnosing a patient who suffers from vibrations in tissues of a throat of the patient when air flows through an airway of the patient. The vibration can result in a loud, raspy noise which may awaken the patient from sleep, awaken others in the patient's surrounding environment, or both. Snoring can be difficult for the patient to detect without assistance from another person (e.g., a health care provider, a bed partner, etc.). Snoring is more common in patients who are overweight, smoke, are back-sleepers, or suffer from congestion, though snoring can occur in other patients as well.

In some aspects, the second device is a continuous positive air pressure machine.

In some aspects, the second device is a mandibular advancement device.

In some aspects, the method 1200 also includes positioning the sleep strip such that an opening in a first layer of the sleep strip lies directly above a mouth of the patient.

In some aspects, the method 1200 also includes affixing the sleep strip to an upper lip and a lower lip of the patient with an adhesive.

In some aspects, the method 1200 also includes covering a mouth of the patient with the sleep strip.

In some aspects, the method 1200 also includes forming a semi-secure seal over the mouth of the patient with the sleep strip.

In some aspects, the step 1203 of the method 1200 includes reducing an incidence of snoring in the patient to a predetermined level relative to an incidence of snoring in the patient prior to any treatment for snoring. In some aspects, reducing an incidence of snoring includes reducing a frequency of snores, a decibel level of snores, or both. In some aspects, the method 1200 also includes reducing an incidence of snoring in the patient relative to treatment without using a sleep strip as disclosed herein. As one example, the method 1200 may include reducing an incidence of snoring in a patient from between 20% and 100%. As a specific example, the method 1200 may also include reducing an incidence of snoring in the patient by between 50% and 90% relative to the incidence of snoring in the patient prior to treatment using the sleep strip disclosed herein. In some aspects, the method 1200 may also include reducing a decibel level of snores of the patient relative to treatment without using a sleep strip as disclosed herein. For example, the method 1200 may also include reducing the decibel level of snores of the patient by between 50% and 90% relative to the incidence of snoring in the patient prior to treatment using the sleep strip disclosed herein.

A method for treating sleep-disordered breathing in a patient is disclosed. The method includes the steps of prior to sleeping, placing a sleep strip on a face of the patient, the sleep strip being configured to maintain lips and/or a jaw of the patient in a closed posture; and disposing a device configured to facilitate stable breathing patterns on a face or in a mouth of the patient. The sleep strip includes a first layer including an adhesive configured to attach the sleep strip to the face of a patient, the first layer having an opening extending therethrough, the opening being located above the lips of the patient, and a mesh layer covering at least the opening of the first layer, the mesh layer being configured to allow air to flow therethrough.

According to one aspect, the method is for treating sleep apnea, and the device is a continuous positive air pressure machine. According to another aspect, the method is for treating sleep apnea, and the device is a mandibular advancement device. According to another aspect, the method is for treating snoring, and the device is a continuous positive air pressure machine. According to another aspect, the method is for treating snoring, and the device is a mandibular advancement device. According to another aspect, the sleep strip is releasably secured only onto the patient's lips. According to another aspect, the mesh layer extends across an entirety of a front side of the first layer. According to another aspect, the mesh layer is located only over the opening of the first layer. According to another aspect, the adhesive is located around an entire border of the opening. According to another aspect, the device is a continuous positive air pressure machine that is disposed on the face of the patient over at least a portion of the sleep strip. According to another aspect, the device is a mandibular advancement device that is disposed on the face of the patient under the sleep strip.

As utilized herein, the terms "approximately," "about," "substantially," and similar terms are intended to have a broad meaning in harmony with the common and accepted usage by those of ordinary skill in the art to which the subject matter of the disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of this disclosure as recited in the appended claims.

The terms "coupled," "connected," and the like are used herein to mean the joining of two members directly or indirectly to one another. Such joining may be stationary (e.g., permanent) or moveable (e.g., removable or releasable). Such joining may be achieved with the two members or the two members and any additional intermediate members being integrally formed as a single unitary body with one another or with the two members or the two members and any additional intermediate members being attached to one another.

References herein to the position of elements (e.g., "top," "bottom," "above," "below," etc.) are merely used to describe the orientation of various elements in the Figures. It should be noted that the orientation of various elements may differ according to other exemplary embodiments and that such variations are intended to be encompassed by the present disclosure.

It is to be understood that although the present invention has been described with regard to embodiments thereof, various other embodiments and variants may occur to those skilled in the art, which are within the scope of the invention. Those skilled in the art will readily appreciate that many modifications are possible (e.g., variations in sizes, structures, shapes and proportions of the various elements, mounting arrangements, use of materials, orientations, etc.) without materially departing from the novel teachings and advantages of the subject matter described herein. For example, the order or sequence of any process or method steps may be varied or re-sequenced according to alternative embodiments. Other substitutions, modifications, changes, and omissions may also be made in the design, operating conditions and arrangement of the various exemplary embodiments without departing from the scope of the present disclosure.

With respect to the use of substantially any plural and/or singular terms herein, those having skill in the art can translate from the plural to the singular and/or from the singular to the plural as is appropriate to the context or application. The various singular/plural permutations may be expressly set forth herein for clarity.

## Claims

1. A system for treating sleep-disordered breathing in a patient, the system comprising:
a sleep strip (40) configured to maintain lips and/or a jaw of the patient in a closed posture, the sleep strip (40) comprising:
a first layer (41) comprising an adhesive configured to attach the sleep strip (40) to a face of a patient, the first layer (41) having an opening (43) extending therethrough, the opening (43) being configured to be located above the lips of the patient when the sleep strip (40) is attached to the face of the patient;
a mesh layer (45); and
a device configured to facilitate stable breathing patterns in the patient during a sleep session;
**characterized in that** the mesh layer (45) extends across an entirety of a surface (42, 44) of the first layer (41) and covers the opening (43), but is configured to allow air to flow therethrough.

2. The system according to claim 1, wherein the sleep strip (40) further comprises at least one release tab (46) configured to facilitate removal of the sleep strip (40) from the face of the patient after the sleep session is completed.

3. The system according to any one of claims 1 or 2, wherein the sleep strip (40) is configured to be releasably secured onto the lips of the patient.

4. The system according to any preceding claim, wherein the sleep strip (40) is configured to be releasably secured only onto the lips of the patient.

5. The system according to any preceding claim, wherein the device is a continuous positive air pressure machine.

6. The system according to any one of claims 1 to 4, wherein the device is a mandibular advancement device.

7. The system according to any preceding claim, wherein the adhesive is located around an entire border of the opening (43).

8. The system according to any preceding claim, wherein the opening (43) defines a cross-sectional area of 125 square millimeters or less.

9. The system according to any preceding claim, wherein:
the first layer (41) further comprises a first surface (44) and a second surface (42), the first surface (44) comprising the adhesive, and the second surface (42) coupled to the mesh layer (45).

10. The system according to any preceding claim, wherein the adhesive comprises a first adhesive strip (52) positioned above the opening (43) and a second adhesive strip (51) positioned below the opening (43).

11. The system according to any preceding claim, wherein the first layer (41) is covered with an adhesive backing in order to keep the adhesive clean and sterile until the time of use.

12. The system according to claim 2, wherein the at least one release tab (46) is connected to the mesh layer (45), wherein the at least one release tab (46) is positioned on a left side of the mesh layer (46) and/or on a right side of the mesh layer (46), and the at least one release tab (46) is free from adhesive.

13. The system according to any preceding claim, wherein the device is separate from the sleep strip (40).

14. The system according to any preceding claim, further comprising an elastic band affixed to the sides of the sleep strip (40) and configured to retain the sleep strip (40) on the mouth and/or lips of the patient by extending the elastic band around the back of the head of the patient.

15. The system according to any preceding claim, wherein the adhesive is configured to securably attach the sleep strip (40) to at least one of the upper vermillion and the lower vermillion of the lips of the patient.

## Patentansprüche

1. System zur Behandlung von schlafbezogener Atmungsstörung bei einem Patienten, wobei das System umfasst:
einen Schlafstreifen (40), der konfiguriert ist, um Lippen und/oder einen Kiefer des Patienten in einer geschlossenen Haltung zu halten, wobei der Schlafstreifen (40) umfasst:
eine erste Schicht (41), die einen Klebstoff umfasst, der konfiguriert ist, um den Schlafstreifen (40) an einem Gesicht eines Patienten anzubringen, wobei die erste Schicht (41) eine sich dadurch erstreckende Öffnung (43) aufweist, wobei die Öffnung (43) konfiguriert ist, um sich oberhalb der Lippen des Patienten zu befinden, wenn der Schlafstreifen (40) am Gesicht des Patienten angebracht ist;
eine Maschenschicht (45); und
eine Vorrichtung, die konfiguriert ist, um stabile Atmungsmuster im Patienten während einer Schlafsitzung zu ermöglichen;
**dadurch gekennzeichnet, dass** sich die Maschenschicht (45) über eine Gesamtheit einer Oberfläche (42, 44) der ersten Schicht (41) erstreckt und die Öffnung (43) bedeckt, aber konfiguriert ist, um zu ermöglichen, dass Luft dadurch strömt.

2. System nach Anspruch 1, wobei der Schlafstreifen (40) ferner mindestens eine Ablöselasche (46) umfasst, die konfiguriert ist, um das Entfernen des Schlafstreifens (40) vom Gesicht des Patienten zu erleichtern, nachdem die Schlafsitzung abgeschlossen ist.

3. System nach einem der Ansprüche 1 oder 2, wobei der Schlafstreifen (40) konfiguriert ist, um lösbar an den Lippen des Patienten befestigt zu werden.

4. System nach einem der vorhergehenden Ansprüche, wobei der Schlafstreifen (40) konfiguriert ist, um lösbar nur an den Lippen des Patienten befestigt zu werden.

5. System nach einem der vorhergehenden Ansprüche, wobei die Vorrichtung eine kontinuierliche Überdruckmaschine ist.

6. System nach einem der Ansprüche 1 bis 4, wobei die Vorrichtung eine Unterkiefervorschubvorrichtung ist.

7. System nach einem der vorhergehenden Ansprüche, wobei sich der Klebstoff um einen gesamten Rand der Öffnung (43) herum befindet.

8. System nach einem der vorhergehenden Ansprüche, wobei die Öffnung (43) eine Querschnittsfläche von 125 Quadratmillimeter oder weniger definiert.

9. System nach einem der vorhergehenden Ansprüche, wobei:
die erste Schicht (41) ferner eine erste Oberfläche (44) und eine zweite Oberfläche (42) umfasst, wobei die erste Oberfläche (44) den Klebstoff umfasst und die zweite Oberfläche (42) an die Maschenschicht (45) gekoppelt ist.

10. System nach einem der vorhergehenden Ansprüche, wobei der Klebstoff einen ersten Klebestreifen (52), der über der Öffnung (43) positioniert ist, und einen zweiten Klebestreifen (51), der unterhalb der Öffnung (43) positioniert ist, umfasst.

11. System nach einem der vorhergehenden Ansprüche, wobei die erste Schicht (41) mit einer Kleberückseite bedeckt ist, um den Klebstoff bis zum Zeitpunkt der Verwendung sauber und steril zu halten.

12. System nach Anspruch 2, wobei die mindestens eine Ablöselasche (46) mit der Maschenschicht (45) verbunden ist, wobei die mindestens eine Ablöselasche (46) auf einer linken Seite der Maschenschicht (46) und/oder auf einer rechten Seite der Maschenschicht (46) positioniert ist und die mindestens eine Ablöselasche (46) frei von Klebstoff ist.

13. System nach einem der vorhergehenden Ansprüche, wobei die Vorrichtung vom Schlafstreifen (40) getrennt ist.

14. System nach einem der vorhergehenden Ansprüche, ferner umfassend ein elastisches Band, das an den Seiten des Schlafstreifens (40) befestigt ist und konfiguriert ist, um den Schlafstreifen (40) am Mund und/oder an den Lippen des Patienten zu halten, indem das elastische Band um der Hinterkopf des Patienten gedehnt wird.

15. System nach einem der vorhergehenden Ansprüche, wobei der Klebstoff konfiguriert ist, um den Schlafstreifen (40) sicher an mindestens einem von dem oberen Zinnoberrot und dem unteren Zinnoberrot der Lippen des Patienten anzubringen.

## Revendications

1. Système destiné au traitement des troubles respiratoires du sommeil chez un patient, le système comprenant :
une bande de sommeil (40) conçue pour maintenir les lèvres et/ou une mâchoire du patient dans une posture fermée, la bande de sommeil (40) comprenant :
une première couche (41) comprenant un adhésif conçu pour attacher la bande de sommeil (40) au visage d'un patient, la première couche (41) possédant une ouverture (43) la traversant, l'ouverture (43) étant conçue pour être située au-dessus des lèvres du patient lorsque la bande de sommeil (40) est attachée au visage du patient ; une couche de maille (45) ; et
un dispositif conçu pour faciliter des motifs de respiration stables chez le patient durant une session de sommeil ;
**caractérisé en ce que** la couche de maille (45) s'étend sur la totalité d'une surface (42, 44) de la première couche (41) et recouvre l'ouverture (43), mais est conçue pour permettre à l'air de s'écouler à travers celle-ci.

2. Système selon la revendication 1, ladite bande de sommeil (40) comprenant en outre au moins une languette de libération (46) conçue pour faciliter le retrait de la bande de sommeil (40) du visage du patient après que la séance de sommeil est terminée.

3. Système selon l'une quelconque des revendications 1 ou 2, ladite bande de sommeil (40) étant conçue pour être fixée de manière amovible aux lèvres du patient.

4. Système selon une quelconque revendication précédente, ladite bande de sommeil (40) étant conçue pour être fixée de manière amovible uniquement sur les lèvres du patient.

5. Système selon une quelconque revendication précédente, ledit dispositif étant une machine à pression d'air positive continue.

6. Système selon l'une quelconque des revendications 1 à 4, ledit dispositif étant un dispositif d'avancement mandibulaire.

7. Système selon une quelconque revendication précédente, ledit adhésif étant situé autour d'un bord entier de l'ouverture (43).

8. Système selon une quelconque revendication précédente, ladite ouverture (43) définissant une aire de section transversale inférieure ou égale à 125 millimètres carrés.

9. Procédé selon une quelconque revendication précédente,
ladite première couche (41) comprenant en outre une première surface (44) et une seconde surface (42), la première surface (44) comprenant l'adhésif, et la seconde surface (42) étant couplée à la couche de maille (45).

10. Système selon une quelconque revendication précédente, ledit adhésif comprenant une première bande adhésive (52) positionnée au-dessus de l'ouverture (43) et une seconde bande adhésive (51) positionnée au-dessous de l'ouverture (43).

11. Système selon une quelconque revendication précédente, ladite première couche (41) étant recouverte d'un support adhésif afin de maintenir l'adhésif propre et stérile jusqu'au moment de l'utilisation.

12. Système selon la revendication 2, ladite au moins une languette de libération (46) étant raccordée à la couche de maille (45), ladite au moins une languette de libération (46) étant positionnée sur un côté gauche de la couche de maille (46) et/ou sur un côté droit de la couche de maille (46), et l'au moins une languette de libération (46) étant exempte d'adhésif.

13. Système selon une quelconque revendication précédente, ledit dispositif étant séparé de la bande de sommeil (40).

14. Système selon une quelconque revendication précédente, comprenant en outre une bande élastique fixée aux côtés de la bande de sommeil (40) et conçue pour retenir la bande de sommeil (40) sur la bouche et/ou les lèvres du patient en étendant la bande élastique autour de l'arrière de la tête du patient.

15. Système selon une quelconque revendication précédente, ledit adhésif étant conçu pour attacher de manière sûre la bande de sommeil (40) à au moins l'un du vermillon supérieur et du vermillon inférieur des lèvres du patient.
